(19) Europäisches Patentamt
European Patent Office
Office européen des brevets

(11) Veröffentlichungsnummer: **0 088 962**
**B1**

(12) **EUROPÄISCHE PATENTSCHRIFT**

(45) Veröffentlichungstag der Patentschrift: **03.10.84**

(51) Int. Cl.³: **C 07 C 51/27**, C 07 C 51/487, C 07 C 51/47, C 07 C 59/125, C 07 C 59/305

(21) Anmeldenummer: **83102128.2**

(22) Anmeldetag: **04.03.83**

(54) **Verfahren zur Herstellung von Polyethylenglykolcarbonsäuren.**

(30) Priorität: **16.03.82 DE 3209434**

(43) Veröffentlichungstag der Anmeldung: **21.09.83 Patentblatt 83/38**

(45) Bekanntmachung des Hinweises auf die Patenterteilung: **03.10.84 Patentblatt 84/40**

(84) Benannte Vertragsstaaten: **AT CH DE FR LI SE**

(56) Entgegenhaltungen:
**EP - A - 0 048 396**
**DE - A - 2 832 949**
**US - A - 2 659 754**
**US - A - 3 632 640**

**HOUBEN-WEYL: "Methoden der Organischen Chemie", 4. Auflage, Band IV/1a, Oxidation, Teil I, 1981, Seiten 649-656, Georg Thieme Verlag, Stuttgart, DE. "A1) Salpetersäure als Oxidationsmittel"**

(73) Patentinhaber: **BASF Aktiengesellschaft, Carl-Bosch-Strasse 38, D-6700 Ludwigshafen (DE)**

(72) Erfinder: **Disteldorf, Walter, Dr., Portugieser Weg 17, D-6706 Wachenheim (DE)**
Erfinder: **Eisfeld, Wolfgang, Dr., Dubliner Sreasse 6, D-6700 Ludwigshafen (DE)**
Erfinder: **Hellbach, Hans, Stettiner Strasse 14, D-6840 Lampertheim (DE)**

**EP 0 088 962 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Diese Erfindung betrifft ein Verfahren zur Herstellung von Polyethylenglykolcarbonsäuren durch Oxidation von Polyethylenglykolen oder ihren Monomethylethern mit Salpetersäure in Gegenwart einer Vanadinverbindung als Katalysator.

Polyethylenglykolsäuren lassen sich bekanntlich durch Oxidation der entsprechenden Polyethylenglykole mit Salpetersäure herstellen. Nach den Angaben der GB-PS Nr. 639491 nimmt man die Oxidation bei Temperaturen von 40 bis 90° C in Abwesenheit oder Gegenwart von Vanadinverbindungen als Katalysator vor, wobei man die Triglykolsäure nach Abdestillieren von Wasser und überschüssiger Salpetersäure aus dem Rückstand isoliert. Man erhält hierbei die Triglykolsäure in unbefriedigender Ausbeute und Reinheit. Bei dem in der DE-OS Nr. 2206862 beschriebenen Verfahren gibt man Stickstoffoxide zum Oxidationsgemisch. Dadurch wird zwar die Ausbeute verbessert, man erhält aber ein Rohprodukt, das zur Gewinnung der reinen Diglykolsäure umkristallisiert werden muss. Ausserdem ist die Einengung der wässerigen, salpetersauren Reaktionslösung in technischem Umfang mit erheblichen Gefahren verbunden. Wird im Vakuum destilliert, so gelangt man zu einer Entwässerung des Reaktionsgemisches und damit zu einer Anreicherung von hochkonzentrierter Salpetersäure im Sumpf, wodurch detonationsfähige Gemische entstehen. Versucht man, die salpetersauren Oxidationsgemische drucklos zu destillieren, so kann man zwar bis zu einer Sumpftemperatur von 150° C die Salpetersäure gefahrlos entfernen, man hat hierbei jedoch hohe Ausbeuteverluste durch Überoxidation hinzunehmen. Ausserdem weist die so erhaltene Rohsäure einen N-Gehalt von ≧ 0,3% auf.

Es wurde nun gefunden, dass man bei der Herstellung von Polyethylenglykolcarbonsäuren, bei der man Polyethylenglykole oder ihre Monomethylether in Gegenwart einer Vanadinverbindung als Katalysator bei höherer Temperatur mit überschüssiger Salpetersäure behandelt und das Reaktionsgemisch destillativ aufarbeitet, erheblich vorteilhaftere Ergebnisse erzielt, wenn man das Reaktionsgemisch vor der Destillation zum Abbau überschüssiger Salpetersäure bei Temperaturen von 60 bis 100° C mit Formaldehyd, Paraformaldehyd, Trioxan oder Ameisensäure behandelt, das dabei erhaltene Gemisch unter Abdestillieren von Wasser ohne Vakuum auf Temperaturen von über 100° C erhitzt und aus dem Rückstand den Katalysator mit Hilfe eines sauren Ionenaustauschers abtrennt.

Nach dem neuen Verfahren lassen sich die Polyethylenglykolsäuren technisch sicher und mit hoher Selektivität unmittelbar in reiner Form herstellen.

Als Ausgangsstoffe verwendet man Polyethylenglykole mit mittleren Molekulargewichten z.B. bis 800, vorzugsweise bis 450, oder Monomethylether von Polyethylenglykolen mit Molekulargewichten bis 300, vorzugsweise bis 200. Der an sich bekannte Reaktionsverlauf sei durch die folgenden Formeln veranschaulicht:

$$HOCH_2CH_2O(CH_2CH_2O)_nCH_2OH \xrightarrow{HNO_3} HOOCCH_2O(CH_2CH_2O)_nCH_2COOH$$

$$CH_3-O(CH_2CH_2O)_mCH_2CH_2OH \xrightarrow{HNO_3} CH_3-O(CH_2CH_2O)_mCH_2COOH$$

wobei n und m die durch die entsprechenden Molgewichte gegebenen Zahlen bedeuten.

Die Oxidation der Polyethylenglykole oder der entsprechenden Monomethylether mit der Salpetersäure führt man im allgemeinen bei Temperaturen von 40 bis 80° C durch. Die Salpetersäure wird im Überschuss angewendet. Ihre Menge beträgt jedoch im allgemeinen nicht mehr als 4 mol/mol Hydroxylgruppe. Vorzugsweise wendet man 2,5 bis 3 mol Salpetersäure pro Mol Hydroxylgruppe an. Zweckmässigerweise wird die Salpetersäure als 30 bis 80%ige wässerige Salpetersäure eingesetzt. Sie wird bei der diskontinuierlichen Arbeitsweise vorzugsweise insgesamt und zusammen mit dem Katalysator vorgelegt.

Zum verzögerungsfreien Starten der Oxidation hat es sich als vorteilhaft erwiesen, vor der Zugabe der Glykolverbindung das zum Einleiten der Reaktion benötigte freie $NO_2$ durch Zugabe von Formaldehyd, Paraformaldehyd oder Trioxan bei Temperaturen von 40 bis 60° C zur vorgelegten Salpetersäure zu erzeugen. Da hierbei die Menge an freiem $NO_2$ 0,1 bis 2, vorzugsweise 0,3 bis 0,9 Gew.-%, bezogen auf die wässerige Salpetersäure, betragen sollte, gibt man, bezogen auf die wässerige Salpetersäure, 0,03 bis 1, vorzugsweise 0,09 bis 0,4 Gew.-% an Formaldehyd, Paraformaldehyd oder Trioxan zur vorgelegten Salpetersäure.

Als Katalysatoren verwendet man Vanadin oder Vanadinverbindungen, die im Reaktionsgemisch Vanadationen bilden können. Geeignet sind z.B. Ammoniumvanadat, Vanadinpentoxid oder Vanadinoxidsulfat, die man zur vorgelegten Salpetersäure gibt. Besonders vorteilhafte Ergebnisse werden erzielt, wenn man Katalysatoren verwendet, die ausser den Vanadinverbindungen noch Kupfer oder Kupferverbindungen enthalten, welche im Reaktionsgemisch Kupferionen bilden können. Geeignete Kupferverbindungen sind z.B. Kupferacetat, -carbonat oder -nitrat. Der Katalysator wird z.B. in einer Menge von 0,003 bis 0,3, vorzugsweise 0,03 bis 0,07 Gew.-% (berechnet auf V und Cu), bezogen auf die wässerige Salpetersäure, eingesetzt. Das g-Atomverhältnis Vanadium zu Kupfer kann z.B. 5:1 bis 1:8 betragen. Besonders günstig hat sich ein g-Atomverhältnis Vanadium zu Kupfer von 1:1 bis 1:4 erwiesen. Man oxidiert zweckmässig bei Normaldruck, wobei man bis zum 85%igen Umsatz eine Temperatur von 60° C nicht überschreiten sollte. Zur Nachoxidation kann dann die Temperatur auf 80° C erhöht werden.

Nach dem erfindungsgemässen Verfahren wird das Reaktionsgemisch vor der Destillation zum Abbau überschüssiger Salpetersäure bei Temperaturen von 60 bis 100° C mit Formaldehyd, Paraformaldehyd, Trioxan oder Ameisensäure behandelt. Pro Mol noch vorhandener Salpetersäure nimmt

man zweckmässig 0,5 bis 1,5, vorzugsweise 0,7 bis 0,9 mol Formaldehyd oder die entsprechende Menge der genannten Polymeren des Formaldehyds oder 0,8 bis 2,4, vorzugsweise 1,2 bis 1,6 mol Ameisensäure. Formaldehyd wird zweckmässig als etwa 25 bis 40%ige wässerige Lösung eingesetzt. Diese Behandlung wird vorzugsweise bei 85 bis 95°C durchgeführt. Die Behandlungsdauer beträgt normalerweise mindestens 30 min, vorzugsweise etwa 1 bis 3 h. Nach dieser Behandlung liegt der Salpetersäuregehalt des Reaktionsgemisches bei Werten unter 1 Gew.-%. Nun wird unter gleichzeitigem Abdestillieren von Wasser ohne Vakuum auf Temperaturen über 100°C, beispielsweise auf 105 bis 110°C, erhitzt, wobei man als Rückstand eine wässerige Säure erhält, die überraschenderweise einen Salpetersäuregehalt von unter 0,1 Gew.-% aufweist. Diese weitgehende Zerstörung der Salpetersäure ist an einem Farbumschlag von blaugrün nach tiefblau zu erkennen, der durch Reduktion des Vanadats zum $VO^{2+}$-Ion bedingt ist.

Nun wird aus dem Konzentrat nach Verdünnen mit Wasser auf eine etwa 50%ige Polyethylencarbonsäure der Katalysator auf an sich übliche Weise mit Hilfe eines sauren Ionenaustauschers abgetrennnt. Hierfür sind z.B. die im Handel erhältlichen sauren Ionenaustauscher, wie ®Dowex 50 WX 8, ®Lewatit S 100 und ®Amberlyst 15 geeignet. Zweckmässigerweise gibt man die flüssigen Konzentrate über eine mit dem Ionenaustauscher gefüllte Säule, wobei der in kationischer Form als $VO^{2+}$ oder $Cu^{2+}$ vorliegende Katalysator auf dem Ionenaustauscher abgeschieden wird. Zweckmässigerweise wird das Austauscherbett mit Wasser nachgewaschen, bis die ablaufende Lösung einen pH von 5 bis 6 aufweist.

Sowohl die vom Katalysator befreite Lösung als auch die durch das Nachwaschen des Ionenaustauschers erhältliche wässerige Fraktion werden nun so weit eingeengt, vorzugsweise durch Abdestillieren des Wassers bei Temperaturen unter 100°C im Vakuum, dass der Restwassergehalt unter 0,5 Gew.-% liegt. Man erhält die Polyethylenglykolsäuren dabei unmittelbar in so hoher Reinheit, dass ein Umkristallisieren nicht erforderlich ist.

Der Katalysator kann von dem Ionenaustauscher mit halbkonzentrierter Salpetersäure eluiert werden und kann nach Aufkonzentration der Lösung ohne Zugabe eines neuen Katalysators für eine erneute Oxidation eingesetzt werden. Selbst nach 10maliger Recyclisierung ist kein Ausbeuteverlust festzustellen.

Man kann das neue Verfahren sowohl diskontinuierlich als auch kontinuierlich durchführen. Hierbei richtet sich die Dosierrate an Polyethylenglykol oder des Monomethylethers nach den Möglichkeiten der Wärmeabfuhr. Durch den Einsatz von weniger als 4 mol Salpetersäure pro Mol Hydroxylgruppe erhält man nach der Alkoholzugabe zunächst eine Oxidationslösung, die bis zu 15% des eingesetzten Alkohols in Form eines Esters der gewünschten Säure enthält. Dieser Ester wird jedoch unter den Reaktionsbedingungen bei Temperaturen von 60 bis 80°C verseift, wobei der freigesetzte Alkohol zur gewünschten Säure aufoxidiert wird. Zu diesem Zeitpunkt ist die Salpetersäurekonzentration in der Oxidationslösung soweit vermindert, dass eine merkliche saure Etherspaltung, selst bei Temperaturanhebung bis auf 80°C, unterbleibt.

Nach dem erfindungsgemässen Verfahren werden die Polyethylenglykolcarbonsäuren bzw. deren Monomethylether in hoher Ausbeute gefahrlos und technisch besonders einfach erhalten. Darüber hinaus ergibt sich aus den beliebigen Wiederverwendungen des Katalysators eine erheblich verminderte Umweltbelastung.

Die als Verfahrensprodukte erhältlichen Polyethylenglykolcarbonsäuren sind z.B. wichtige Hilfsmittel in der Textilindustrie oder als Lösungsmittel für Farbstoffe und als Zwischenprodukte für die Herstellung von Fungiziden und wässerigen Drahtlacken geeignet.

*Beispiele 1 bis 7*

In einem Rührbehälter aus salpetersäurebeständigem Material, der mit Heiz- und Kühlvorrichtung versehen ist, wurden 3500 Teile 63%ige Salpetersäure und der in der Tabelle genannte Katalysator vorgelegt. Man erwärmte auf 55°C und gab unter Rühren 19 Teile einer 30%igen wässerigen Formaldehydlösung (= 0,16 Gew.-% Formaldehyd, bezogen auf die 63%ige Salpetersäure) hinzu, wobei sich die Lösung durch $NO_2$-Bildung dunkelbraun verfärbte. Über einen Zeitraum von 4 bis 6 h wurde das Polyethylenglykol bzw. der Monomethyleter so zugegeben, dass eine Temperatur von 50 bis 55°C eingehalten wurde. Das Reaktionsgemisch wurde nach der Zugabe des Glykols 1 h bei 60°C und 3 h bei 70°C gehalten. Zur Zerstörung der Salpetersäure wurden dann bei 90 bis 95°C innerhalb von 1 bis 2 h 0,7 mol Formaldehyd pro Mol Salpetersäure im Oxidationsaustrag (Mengenangaben s. Tabelle) gleichmässig zugegeben. Danach wurde noch 1 h unter Rückfluss gerührt. Dann wurde zunächst bei Normaldruck bis zu einer Sumpftemperatur von 105°C Wasser abdestilliert, wobei man eine dunkelblaue Lösung mit einem Restgehalt an Salpetersäure von unter 0,1 Gew.-% erhielt.

Diese Lösung wurde nun mit soviel Wasser ergänzt, dass eine ca. 50%ige Polyethylencarbonsäure vorlag, die über eine mit 100 g des sauren Ionenaustauschers ®Dowex 50 WX 8 (gerechnet trocken)gefüllten Säule gegeben wurde. Anschliessend wurden von der Säule Reste an Polyethercarbonsäure heruntergewaschen, bis das Waschwasser einen pH von 5 bis 6 aufwies. Zur Rückgewinnung des Katalysators wurde der Ionenaustauscher mit 10%iger Salpetersäure so lange gewaschen, bis die ablaufende Salpetersäure farblos war. Diese zurückgewonnene Salpetersäure wurde destillativ aufkonzentriert und als Katalysatorlösung in die Oxidation zurückgeführt.

Die nach der Katalysatorabtrennung über den Ionenaustauscher erhaltene Lösung inklusive Waschwasser wurde bei 50 mbar und 70°C so lange entwässert, bis der Restwassergehalt <0,5%

betrug. Es wurde eine jeweils sehr reine Polyethylenglykolcarbonsäure erhalten.

In der Tabelle sind die erzielten Ausbeuten in Gewichtsprozent, bezogen auf eingesetzten Alkohol, die Säurezahlen sowie der Glykol- und der Oxalsäuregehalt im Endprodukt angegeben.

*Tabelle*

| Beispiel Nr. | Verwendeter Glykol (g) | Mol $HNO_3$ pro Mol HO-Gruppe | Katalysator (g) | Wässeriger Formaldehyd 30%ig (g) | Polyethylenglykolcarbonsäure | | | |
|---|---|---|---|---|---|---|---|---|
| | | | | | Ausbeute (Gew.-%) | Säurezahl | Glykolsäure (Gew.-%) | Oxalsäure (Gew.-%) |
| 1 | Diglykol 742 | 2,5 | 3,85 $Cu(Ac)_2 \cdot H_2O$ 1,75 $NH_4VO_3$ | 575 | 117 | 834 | <0,1 | <0,1 |
| 2 | Triglykol 1050 | 2,5 | 3,8 $Cu(Ac)_2 \cdot H_2O$ 1,75 $NH_4VO_3$ | 534 | 110 | 625 | 0,1 | <0,1 |
| 3 | Pluriol-E 200[1] 1273 | 2,75 | 3,85 $Cu(Ac)_2 \cdot H_2O$ 1,75 $NH_4VO_3$ | 802 | 110 | 500 | <0,1 | <0,1 |
| 4 | Pluriol-E 300[1] 1909 | 2,75 | 3,85 $Cu(Ac)_2 \cdot H_2O$ 1,75 $NH_4VO_3$ | 472 | 105 | 350 | 0,1 | <0,1 |
| 5 | Pluriol-E 400[1] 2545 | 2,75 | 3,85 $Cu(Ac)_2 \cdot H_2O$ 1,75 $NH_4VO_3$ | 600 | 106 | 270 | 0,2 | <0,1 |
| 6 | Methyldiglykol 1448 | 2,9 | 3,85 $Cu(Ac)_2 \cdot H_2O$ 1,75 $NH_4VO_3$ | 745 | 103 | 413 | 0,3 | <0,1 |
| 7 | Methyltriglykol 1980 | 2,9 | 3,85 $Cu(Ac)_2 \cdot H_2O$ 1,75 $NH_4VO_3$ | 486 | 102 | 310 | 0,3 | <0,1 |

[1] Pluriol-E 200, 300 oder 400 = Polyethylenglykol mit dem mittleren Molekulargewicht 200, 300 oder 400.

## Patentansprüche

1. Verfahren zur Herstellung von Polyethylenglykolcarbonsäuren, bei der man Polyethylenglykole oder ihre Monomethylether in Gegenwart einer Vanadinverbindung als Katalysator bei höherer Temperatur mit überschüssiger Salpetersäure behandelt und das Reaktionsgemisch destillativ aufarbeitet, dadurch gekennzeichnet, dass man das Reaktionsgemisch vor der Destillation zum Abbau überschüssiger Salpetersäure bei Temperaturen von 60 bis 100°C mit Formaldehyd, Paraformaldehyd, Trioxan oder Ameisensäure behandelt, das dabei erhaltene Gemisch unter Abdestillieren von Wasser ohne Vakuum auf Temperaturen von über 100°C erhitzt und aus dem Rückstand den Katalysator mit Hilfe eines sauren Ionenaustauschers abtrennt.

2. Verfahren nach Anspruch 1, dadurch gekennzeichnet, dass man einen Katalysator verwendet, der Vanadat- und Kupferionen enthält.

3. Verfahren nach Anspruch 1, dadurch gekennzeichnet, dass man die Salpetersäure und den Katalysator vorlegt und vor der Zugabe des Polyethylenglykols oder des Monomethylethers bei Temperaturen von 40 bis 60°C 0,03 bis 1 Gew.-%, bezogen auf Salpetersäure, an Formaldehyd, Paraformaldehyd oder Trioxan zur Salpetersäure gibt.

## Claims

1. A process for the preparation of polyethylene glycol carboxylic acids by treating polyethylene glycols or their monomethyl ethers with excess nitric acid in the presence of a vanadium compound as catalyst, at elevated temperature, and working up the reaction mixture by distillation, wherein the reaction mixture, prior to the distillation, is treated with formaldehyde, paraformaldehyde, trioxane or formic acid at a temperature of from 60 to 100°C to decompose the excess nitric acid; the resulting mixture is heated to a temperature of more than 100°C, while distilling off water without the application of a vacuum; and the catalyst is separated from the residue with the aid of an acidic ion exchanger.

2. A process as claimed in Claim 1, wherein a catalyst containing vanadate or copper ions is used.

3. A process as claimed in Claim 1, wherein the nitric acid and the catalyst are initially charged and,

prior to the addition of the polethylene glycol or monomethyl ether, from 0.03 to 1.00% by weight, based on nitric acid, of formaldehyde, paraformaldehyde or trioxane is added to the nitric acid at a temperature of from 40 to 60°C.

## Revendications

1. Procédé de préparation d'acides polyéthylèneglycolcarboxyliques, en traitant avec de l'acide nitrique en excès des polyéthylèneglycols ou leurs éthers monométhyliques, en présence d'un composé du vanadium comme catalyseur, à température élevée, et en achevant ensuite le traitement du mélange réactionnel par distillation, caractérisé en ce que l'on traite le mélange réactionnel, avant la distillation, avec du formaldéhyde, du paraformaldéhyde, du trioxanne ou de l'acide formique, à des températures de 60 à 100°C, pour décomposer l'acide nitrique en excès, en ce qu'on chauffe le mélange ainsi obtenu à des températures supérieures à 100°C sans faire le vide, avec élimination de l'eau par distillation, et en ce qu'on sépare du résidu le catalyseur au moyen d'un échangeur d'ions acide.

2. Procédé suivant la revendication 1, caractérisé en ce que l'on utilise un catalyseur, qui contient des ions vanadate et des ions cuivre.

3. Procédé suivant la revendication 1, caractérisé en ce que l'on introduit l'acide nitrique et le catalyseur dans le récipient de réaction et en ce que, avant l'addition du polyéthylèneglycol ou de l'éther monométhylique, on ajoute à l'acide nitrique, à des températures de 40 à 60°C, 0,03 à 1% en poids de formaldéhyde, paraformaldéhyde ou trioxanne, par rapport à l'acide nitrique.